# EUROPEAN PATENT APPLICATION

(11) **EP 0 142 057 A2**
(43) Date of publication of application: **22.05.1985**
(21) Application number: 84112495.1
(22) Date of filing: 17.10.1984
(51) Int. Cl.: C07D 513/04, A61K 31/425

(54) **Thiazoloquinazoline derivatives, their preparation and pharmaceutical compositions containing same**

(30) Priority: 04.11.1983 US 548758
(71) Applicant: F. HOFFMANN-LA ROCHE AG, 4002 Basel (CH)
(72) Inventor: Carson, Matthew, Nutley, N.J. 07110 (US); LeMahieu, Ronald Andrew, North Caldwell, N.J. 07006 (US); Tilley, Jefferson Wright, North Caldwell, N.J. 07006 (US)
(74) Representative: Kellenberger, Marcus, Dr.

(57) **Abstract**

Compounds of the formula 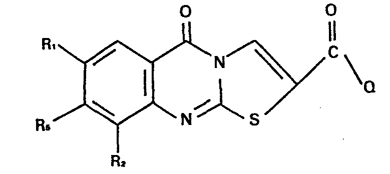 wherein R₁ is hydrogen, lower alkyl, lower cycfoalkyl, lower alkoxy, hydroxy frafogen lower alkylthio lower alkylsulfirryl lower alkylsulfonyl, di-lower alkyl- NiCH₂)nO- or 2-hydroxyethoxy; n is 2 to 7; R₂ is hydrogen lower alkyl or lower alkoxy; R₅ is hydrogen or lower alkyl; provided that if R₂ is otherthan hydrogen, R₅ is hydrogen; O is one of the groups 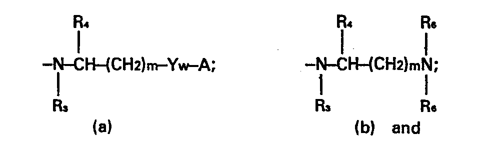 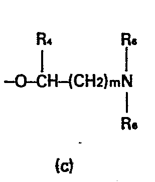 wherein R₃ is hydrogen or methyl; R₄ is hydrogen or lower alkyl; R₅ is lower alkyl; m is 1 to 7; w is zero or one; Y is-NH-; -NH-CH₂; -0-; or -S.; and A is an unsubstituted or substituted heterocyclic radical; and pharmaceutically acceptable acid addition salts thereof are described. The compounds of formula I are useful as anti-allergic agents as well as agents for the treatment of vascular disorders.

## Description

The present invention relates to thiazolo[2,3-b]quinazolines of the general formula 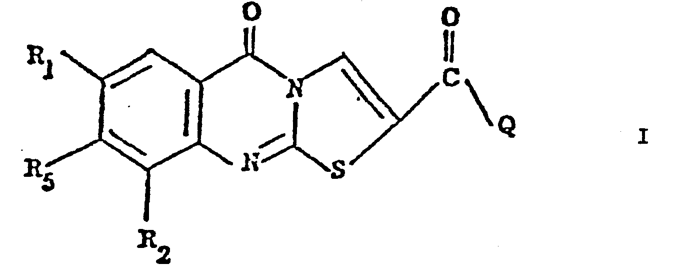 wherein R₁ is hydrogen. lower-alkyl, lower cycloalkyl, lower alkoxy, hydroxy, halogen, lower alkylthio. lower alkylsulfinyl, lower alkylsulfonyl, di-lower alkyl--N(CH₂)ₙO- or 2-hydroxyethoxy; n is 2 to 7; R₂ is hydrogen, lower alkyl or lower alkoxy: R₅ is hydrogen or lower alkyl: provided that if R₂ is other than hydrogen, R₅ is hydrogen; Q is one of the groups 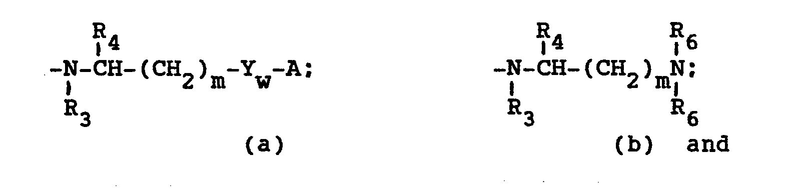 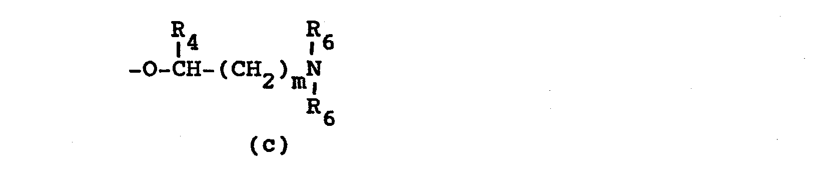 wherein R₃ is hydrogen or methyl: R₄ is hydrogen or lower alkyl; R₆ is lower alkyl: m is 1 to 7: w is zero or one: Y is -NH-: -NH-CH₂-; -0-: or -S.: and A is an unsubstituted or substituted heterocyclic radical: and pharmaceutically acceptable acid addition salts thereof.

As used herein, the term "lower alkyl" denotes a straight or branched-chain saturated hydrocarbon containing 1 to 7 carbon atoms, for example, methyl, ethyl, propyl, isopropyl, butyl, t-butyl. neopentyl, pentyl, heptyl and the like. The term "lower alkoxy" denotes an alkoxy group in which the lower alkyl group is as described above, for example, methoxy, ethoxy, propoxy, pentoxy, and the like. The term "lower alkylthio" denotes an alkylthio group in which the lower alkyl group is as described above, for example, methylthio, ethylthio, propylthio, pentylthio, and the like. The term "lower alkylsulfinyl" denotes an alkylsulfinyl group in which the lower alkyl group is as described above, for example, methylsulfinyl. ethylsulfinyl, and the like. The term "lower alkylsulfonyl" denotes an alkylsulfonyl, group in which the lower alkyl group is as described above, for example, ethylsulfonyl. and the like. The term "halogen" denotes the four halogens bromine, chlorine, fluorine and iodine. Exemplary of di-(lower)-alkyl--N-(CH₂)ₙ-O-groups are dimethylaminoethoxy, diethyl- aminoethoxy, dipropylaminoethoxy, diisopropylaminobutoxy, dibutylaminoethoxy, dipentylaminoethoxy, or the like. The term "lower cycloalkyl" denotes a cyclic hydrocarbon containing 3 to 7 carbon atoms, for example, cyclopropyl, cyclobutyl. cyclopentyl, cyclohexyl and cycloheptyl.

The term "heterocyclic radical" denotes a 5- or 6-membered heterocyclic ring unsubstituted or substituted, especially hetero-aromatic rings which contain 1 to 3, or particularly 1 or 2, hetero-atoms which may be the same or different. Nitrogen, oxygen and sulfur are the preferred hetero--atoms.

Examples of heterocylic radicals are pyridinyl, pyrimidinyl, imidazolyl, furyl, thiazolyl, oxazolyl, pyrazinyl, quinolinyl, isoquinolinyl, quinazolinyl, 1.2,4-triazinyl, benzimidazolyl and pyridazinyl. Preferred examples of heterocyclic radicals are pyridinyl, imidazolyl and pyrimidinyl.

In a preferred aspect, the invention comprises compounds of formula I wherein R₁ is lower alkyl, most preferably isopropyl; R₂ is hydrogen or lower alkyl; R₃ is hydrogen or methyl; R₄ is hydrogen and R₅ is hydrogen; and A is -2, -3 or -4-pyridinyl or 1-imidazolyl.

Preferred compounds of formula I are:
N-Methyl-7-(1-methylethyl)-5-oxo-N-[4-(3-pyridinyl)-butyl]-5H-thiazolo[2.3-b]quinazoline-2-carboxamide:
7-(1-methylethyl)-5-oxo-N-[2-(4-pyridinylthio)ethyl]-5H-thiazolo[2,3-b]quinazoline-2-carboxamide; and
7-(l-methylethyl)-5-oxo-N-[3-pyridinyloxy)butyl]-5H-thiazolo[2.3-b]quinazoline-2-carboxamide.

Particularly preferred compounds of formula I are:
7-(1-Methylethyl)-5-oxo-N-[4-(3-pyridinyl)butyl]-5H-thiazolo[2,3-b]quinazoline-2-carboxamide;
7,9-bis(1-methylethyl)-5-oxo-N-[4-(3-pyridinyl)butyl]-5H-thiazolo[2,3-b]quinazoline-2-carboxamide; and
N-[4-(1H-imidazol-1-yl)butyl]-7-(1-methylethyl)-5-oxo-5H-thiazolo[2.3-b]quinazlline-2-carboxamide.

The compounds of formula I and their pharmaceutically acceptable acid addition salts can be manufactured in accordance with the present invention by a process which comprises

### a) reacting an acid chloride of the general formula

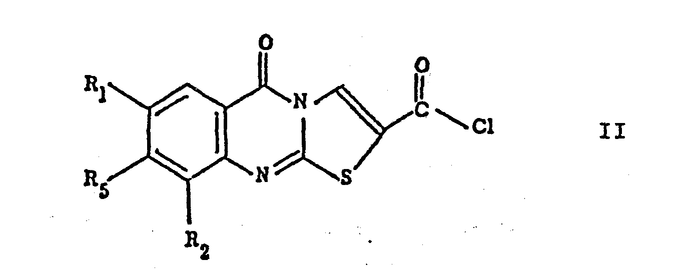

wherein R₁, R₂ and R₅ are as previously described, with a compound of the general formula 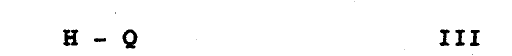 wherein Q is as previously described, or

### b) for the manufacture of a compound of formula I wherein Q is the group (a) or (b), reacting an acid of the general formula

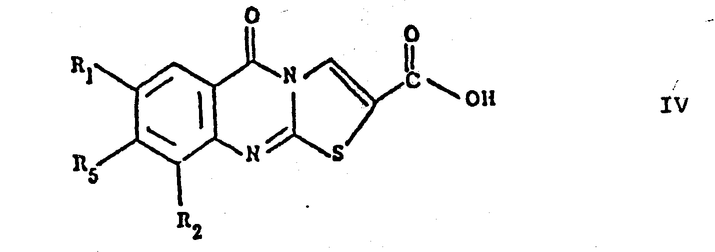

wherein R₁, R₂ and R₅ are as previously described, with a compound of the general formula

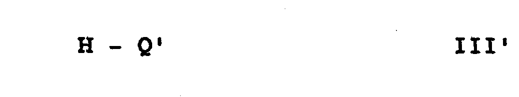

wherein Q' is the group (a) or (b), and, if desired, converting a compound obtained into a pharmaceutically acceptable acid addition salt.

More specifically, the compounds of formula I and their pharmaceutically acceptable acid addition salts as well as various intermediates therefor can be prepared as illustrated hereinbelow in more detail. 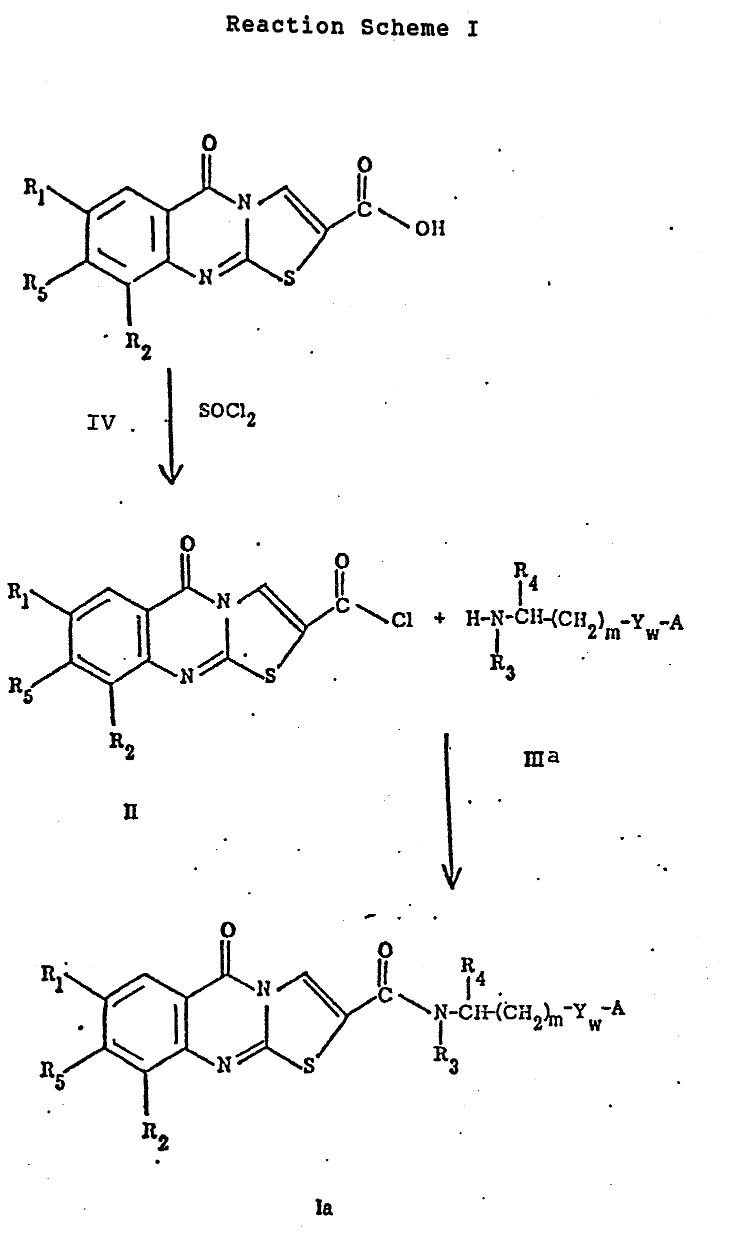 wherein R₁, R₂, R₃, R₄, R₅, m, w, Y and A are as previously described herein.

In Reaction Scheme I, one process for converting the acids of formula IV to the desired end products of formula Ia is shown. In this process an acid of formula IV is converted to the corresponding acid chloride II by reaction with thionyl chloride at elevated temperatures, most preferably at reflux and then treated with an amine of formula IIIa to yield the desired end product of formula Ia. The reaction of the acid chloride with the amine of formula IIIa may be conveniently carried out in the presence of an inert solvent such as dimethylformamide, or an aromatic hydrocarbon such as toluene. A temperature in the range of from about 0°C to the reflux temperature of the medium may be employed, the specific temperature in each case being selected depending upon the nature of the amine reactant. Most preferably anhydrous conditions are utilized. 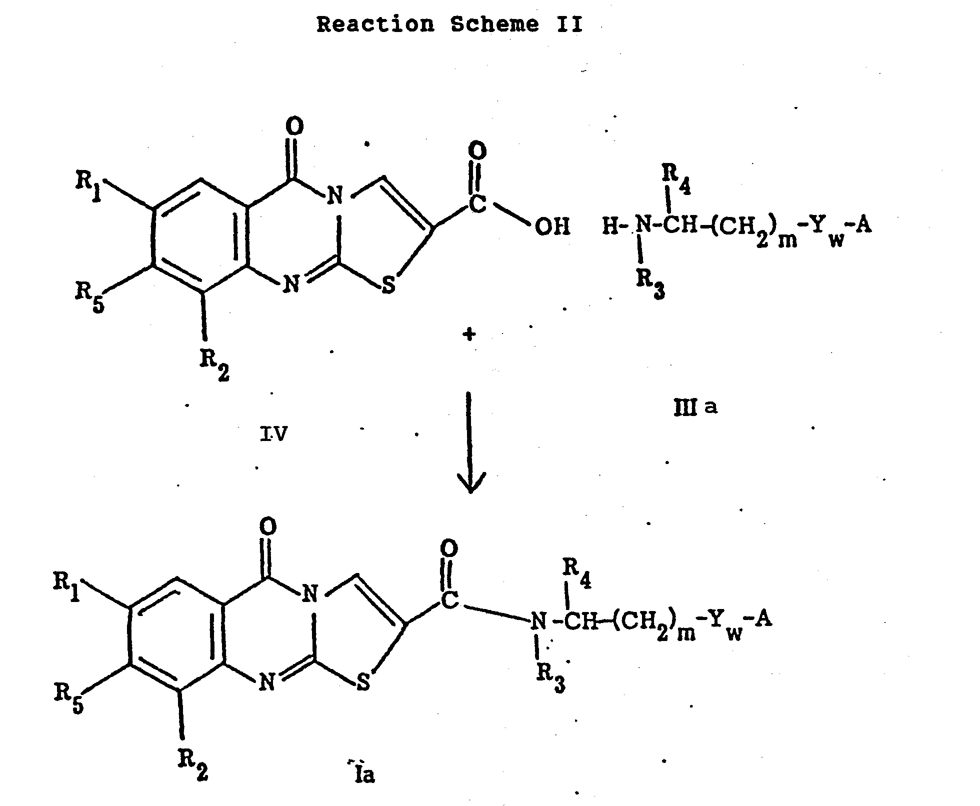 wherein R₁. R₂, R₃, R₄, R₅, m, w, Y and A are as previously described herein.

In Reaction Scheme II, an acid of formula IV can be directly coupled to the desired amines of formula IIIa by utilizing the diphenylphosphoryl azide method. In Reaction Scheme II, the reactants are dissolved in a suitable inert solvent, e.g. anhydrous dimethylformamide with cooling to a temperature of about -5 to about -20°C. The diphenylphosphoryl azide is added dropwise and then a trialkylamine, preferably triethylamine, is added. Workup of the reaction mixture and isolation of the desired end products of formula Ia is carried out in a manner known per se.

Acids of formula IV are known compounds and are disclosed in U.S. Patent No. 4,168,380, U.S. Patent No. 4,282,360 and in R.A. LeMahieu, M. Carson, A.F. Welton, H.W. Baruth and B. Yaremko, J. Med. Chem. 26, 107 (1983).

The acids of formula IV can be conveniently prepared by condensation of the appropriate anthranilic acid with 2-chloro-5-carbomethoxythiazole in the presence of an organic acid, preferably a lower alkanoic acid such as most preferably formic acid, and an inert organic solvent such as an alkoxy alkanol solvent preferably 2-methoxyethanol. The reaction is conveniently carried out at elevated temperatures, preferably at the reflux temperature of the reaction mixture. The resultant esters can be hydrolyzed with sodium hydroxide to the acids of formula IV.

Exemplary compounds of formula IV are:
7-(l-Methylethyl)-5-oxo-5H -thiazolo[2.3-b]quinazoline--2-carboxylic acid: 7,9-bis(1-methylethyl)-5-oxo -5H-thia- zolo[2,3-b]quinazoline-2-carboxylic acid; 7-methoxy-5-oxo--5H-thiazolo[2.3-b]quinazoline -2-carboxylic acid: 7-methylthio-5-oxo-5H-thiazolo[2.3-b]quinazoline -2-carboxylic acid; and 7-methylsulfinyl-5-oxo-5H-thiazolo[2,3-b]quinazoline -2-carboxylic acid.

In Reaction Scheme III, a process for converting the acids of formula IV to the desired end products of formula Ib which are N,N-dialkylaminoalkyl carboxamides is shown. In this process an acid of formula IV is converted to the corresponding acid chloride of formula II as described above. Said acid chloride is then reacted with an appropriate N,N-dialkyl-alkylenediamine in a nonaqueous inert solvent, such as toluene, at reflux for about 1 to 5 hours to yield the desired carboxamide. Alternatively, an end product of formula Ib can be prepared by reacting an acid of formula IV with an N,N-dialkyl-alkylenediamine in accordance with the diphenylphosphoryl azide method described above. 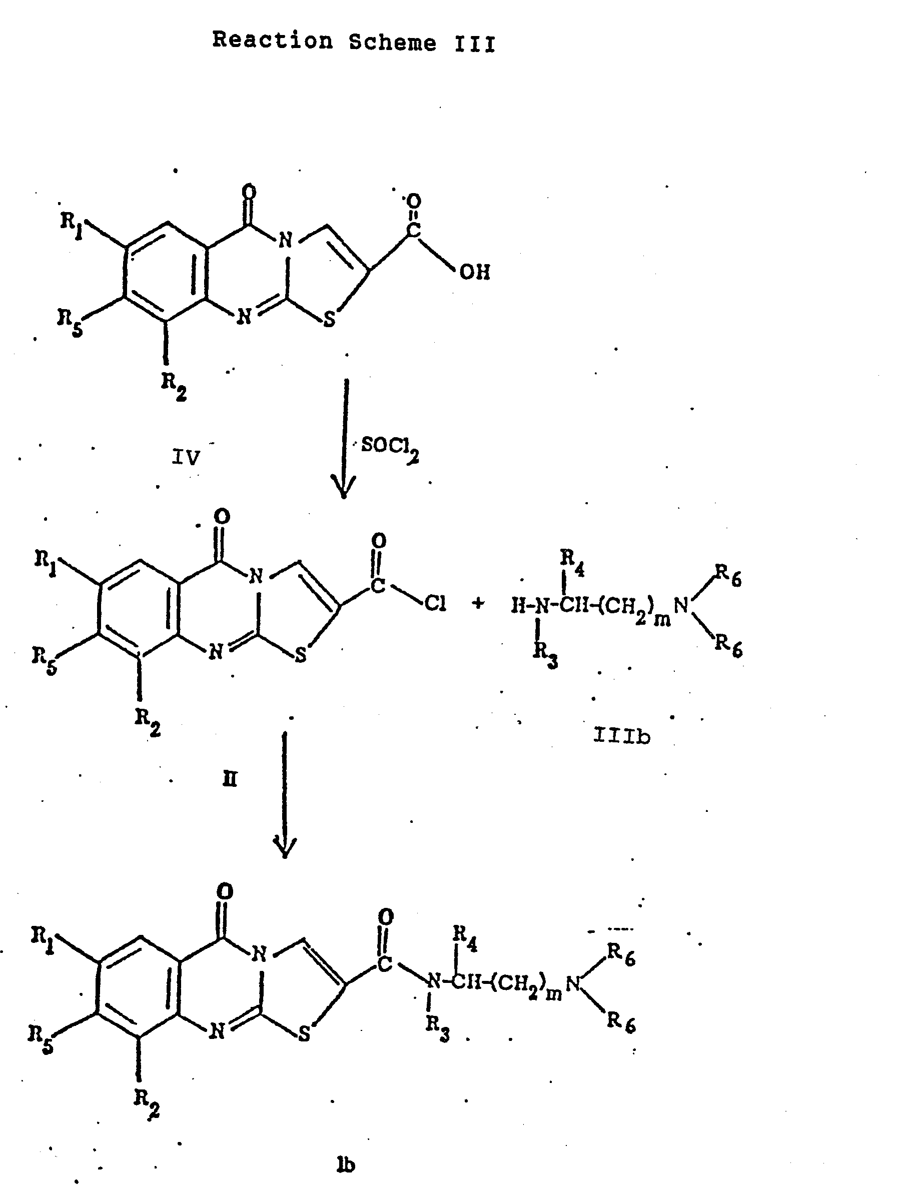 wherein R₁, R₂, R₃, R₄, R₅, R₆ and m are as previously described herein. 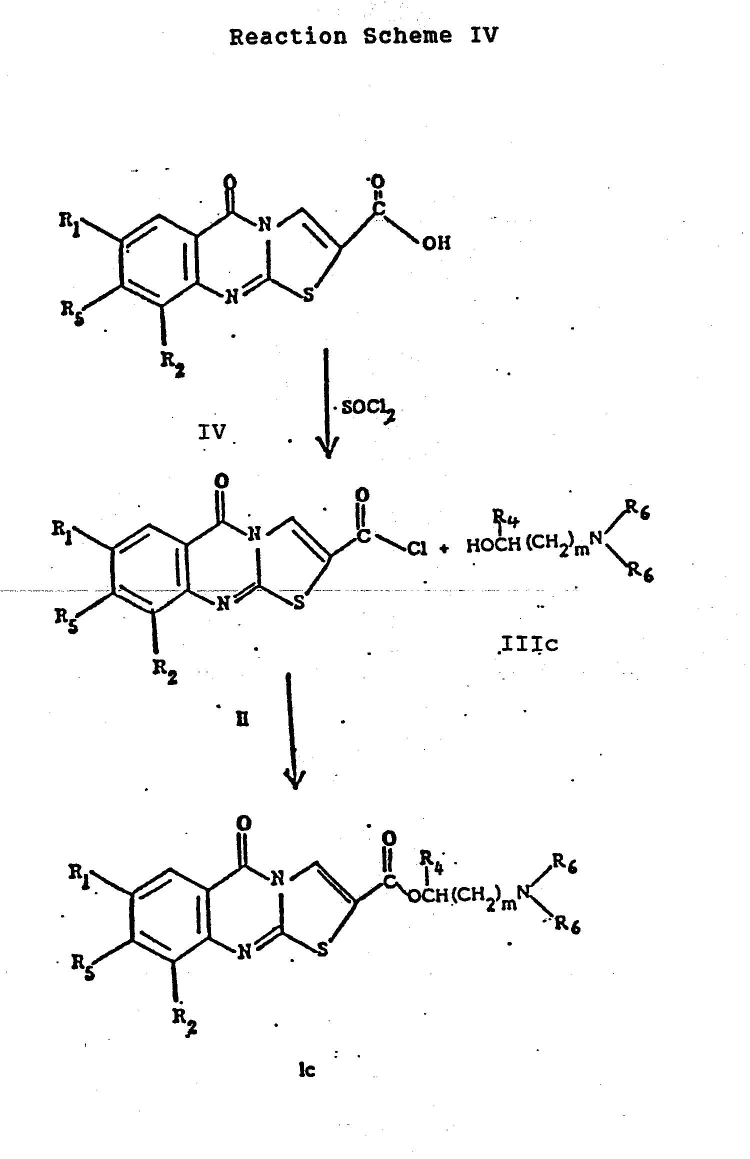 wherein R₁, R₂, R₄, R₅, R₆ and m are as previously described herein.

In Reaction Scheme IV, a process for converting the acids of formula IV to the desired end products of formula Ic which are esters is shown. In this process an acid of formula IV is converted to the corresponding acid chloride of formula II as described above. Said acid chloride is then reacted with an N.N-dialkylaminoalkanol in an inert nonaqueous solvent, such as anhydrous toluene at about room temperature for about 2 to about 4 days to yield the desired ester of formula Ic.

Amines of formula IIIa which fall into the general class of 2- or 3-pyridine-l-alkanamines can be conveniently prepared from corresponding 2- or 3-pyridine-l-alkanenitriles by hydrogenation in a polar solvent such as methanol or ethanol over Raney Cobalt at high temperatures and pressures. This reaction is followed by known work-up procedures for isolating the resultant 2- or 3-pyridine-l-alkanamines. The starting nitriles that is, the 2- or 3-pyridine-l-alkanenitriles are prepared by first passing an inert gas such as argon or neon through a solution of 3-halopyridine and the appropriate alkynyl nitrile in dry triethylamine to which are added small amounts of bis(triphenylphosphine)-palladium dichloride and cuprous iodide. Then work up and isolation of the pyridine alkynylnitrile appropriate for the next step is conducted. In example 18 are contained specific procedures for preparation of 3-pyridine-l-hexanamine.

Amines of formula IIIa which fall into the general class of aryloxyalkanamines can be conveniently prepared by the displacement of the halide from an appropriate haloalkylni- trile with an appropriate arylhydroxy compound in the presence of a reagent such as sodium hydride. This reaction is carried out in a polar nonaqueous solvent such as dimethylformamide at an initial temperature of about 0°C to about room temperature to form the resultant aryloxyalkylnitrile which is then reduced to the corresponding aryloxyalkylamine by treatment with an appropriate reducing agent such as lithium aluminum hydride at a temperature of about 0°C to reflux. Work up and isolation of the desired aryloxyalkylamine are carried out in a usual manner. In Example 9 are contained specific procedures for making 4-(3-pyridinyl- oxy)-l-butanamine.

Amines of formula IIIa which fall into the general class of arylthioalkanamines can be conveniently prepared by reacting an appropriate mercaptoalkanamine with an appropriate halo-substituted aryl compound in a polar protic solvent such as propanol or more preferably ethanol under an inert atmosphere such as nitrogen or more preferably argon, at approximately 0°C to about room temperature for a period of about 1 to about 4 hours. The reaction mixture is then worked up in the usual manner to isolate the desired arylthio- alkanamine.

Amines of formula IIIa which fall into the general class of (1H-imidazol-1-yl)alkanamines are conveniently prepared by hydrogenating an appropriate (1H-imidazol-1-yl)-alkani- trile, with a catalyst, preferably Raney-Cobalt. in a polar solvent, such as, for example, a mixture of triethylamine and methanol, under conditions of high temperature 80-120°C, and high hydrogen pressure (about 800 to about 1200 lbs. hydrogen pressure). The result is the desired (lH-imidazol--1-yl)alkanamine. Said (1H-imidazol-1-yl)alkanamine can be isolated by usual procedures such as evaporation of solvent.

The N(-aryl)-l. 2(or 3)-alkandiamines of formula IIIa are known compounds or can be prepared according to known procedures.

Examples of amines of formula IIIa include:
2-pyridine-l-butanamine:
3-pyridine-l-butanamine:
N-methyl-3-pyridine-l-butanamine:
4-(3-pyridyloxy)-1-butanamine;
(1H-imidazol-1-yl)-1-butanamine;
2-(4-pyridylthio)-1-ethanamine;
3-pyridine-1-hexamine; and
N-(3-pyridinylmethyl)-1.2-ethandiamine.

The N,N'-dialkylalkylenediamines of formula IIIb are known compounds or can be prepared according to known procedures. Examples of said compounds are:
N,N-diethylethylenediamine: and
N.N-dipropylpropylenediamine.

The N,N-dialkylamino alkanols of formula IIIc are known compounds or can be prepared according to known procedures.

Examples of said compounds are:
N,N-diethylamino ethanol: and
N,N-dipropylamino propanol.

The compounds of formula I above are basic compounds which form acid addition salts with inorganic or organic acids. More particularly, the compounds of formula I form, with pharmaceutically acceptable organic or inorganic acids, pharmaceutically acceptable acid addition salts, for example, hydrohalides such as hydrochloride, hydrobromide or hydroiodide, other mineral acids salts such as sulfate, nitrate, phosphate or the like, alkyl and mono-aryl sulfonates such as ethanesulfonate, toluenesulfonate, benzenesulfonate, or the like, other organic acid salts such as acetate, tartrate, maleate, citrate, benzoate, salicylate, ascorbate, or the like. Non-pharmaceutically acceptable acid addition salts of the compounds of formula I above can be converted into pharmaceutically acceptable acid addition salts via conventional metathetic reactions whereby the non-pharmaceutically acceptable anion is replaced by a pharmaceutically acceptable anion; or alternatively, by neutralizing the non--pharmaceutically acceptable acid addition salt and then reacting the so-obtained free base with a reagent yielding a pharmaceutically acceptable anion.

The compounds of formula I, including their salts, are antagonists of bronchoconstriction induced by Slow reacting substance of anaphylaxis (SRS-A) as well as inhibitors of its synthesis. Accordingly the compounds of formula I including their salts, are useful for the treatment of certain allergic conditions such as asthma. In addition, the compounds of formula I, including their salts, inhibit the production of thromboxane A₂ through the inhibition of thromboxane synthase. Accordingly, the compounds of formula I, including their salts, are useful as agents for the treatment of allergic conditions which include skin afflictions, hay fever, chronic bronchitis, obstructive airways diseases such as asthma, allergic conditions of the eye, and allergic conditions of the eye, and allergic conditions of the gastrointestinal tract, such as food allergies and the treatment of vascular disorders and arrhythmias or the like.

The useful antiallergic activity of the compounds of formula I including their salts, can be demonstrated in vitro and in warm-blooded animals utilizing standard procedures. Exemplary of such procedures are:

### (a) Guinea Pi^{q} Ileum, In Vitro

The guinea pig ileum bioassay system has been described by Orange and Austen, Adv. Immunol. 10: 104-144 (1959). A 1.5 cm segment is removed from animals weighing 300-400 g and suspended in an organ bath containing 10 ml of Tyrodes solution with 10⁻⁶ M atropine sulfate and 10⁻⁶ M pyrilamine maleate. The bath is maintained at 37°C and aerated with a mixture of 95% oxygen and 5% carbon dioxide. The SRS-A utilized in this screen is obtained by challenging chopped lung fragments from actively sensitized guinea pigs with egg albumin in vitro. A dose-response curve to SRS-A challenge is established for the ileum. The dose of SRS-A with gives 50% of the maximal contraction (EC₅₀) is then used for subsequent challenge. The drug concentration which inhibits, by 50%, the SRS-A-induced constriction of the guinea pig ileum is determined. In this bioassay system the standard SRS-A antagonist, 7-[3-(4-acetyl-3-hydroxy₋2-pro- pylphenoxy)-2-hydroxypropoxy] -4-oxo-8-propyl-4H-l-benzopyran-2-carboxylic acid, has an IC₅₀ of 3.5 x 10⁻⁸M. Results obtained with representative compounds of the present invention in this assay are summarized hereafter in Table I and III.

### (b) Guinea ^{p}i^{q} Bronchoconstriction, In Vivo

Male guinea pigs (Hostley strain) weighing 300 to 450 grams are anesthetized with urethane (2 g/kg) intraperitoneally and a polyethylene cannula is inserted into the jugular vein for intravenous drug administration. Tracheal pressure is recorded from a cannula inserted in the trachea and connected to a Statham pressure transducer. Respiration is paralyzed with succinyl choline (1.2 mg/kg, i.v.) and the animals are mechanically respirated (Howard rodent respirator) at 40 breaths/minute and 2.5 ml tidal volume. Two minutes thereafter, propranolol (0.1 mg/kg, i.v.) is administered. Five minutes later, the animals are pretreated intravenously for 30 seconds (at 10 mg/kg) with test drug or control vehicle. The animals are subsequently challenged with a maximally constrictory dose of leukotriene E₄ also administered intravenously. The change (cm H₂0) between pre and peak ventilatory pressure readings is averaged for three control animals and five drug treated animals. The percent inhibition is calculated from the following formula:

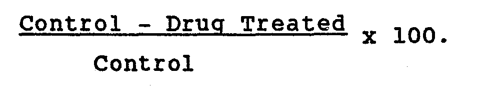

For determination of oral activity spontaneously breathing animals are pretreated orally with drugs (100 mg/kg) for 2 hours (at 100 mg/kg) prior to challenge with leukotriene E₄. 7-[2-(4-Acetyl-3-hydroxy-2-propylphenoxy) -2-hydroxy- propoxy]-4-oxo-8-propyl-4H-1-benzopyran-2-carboxylic acid elicits a 98% inhibition at 10 mg/kg, i.v., but is orally inactive in this test. Representative compounds of the present invention, i.e., 7-(1-methylethyl)-5-oxo-N-[4-(3-pyridinyl)butyl] -5H-thiazolo[2.3-b]quinazoline-2-carboxamide and N-[4-(lH-imidazol-1-yl)butyl]-7-(l-methylethyl)-5-oxo -5H-thiazolo[2,3-b]quinazoline-2-carboxamide dihydro-chloride 0.45 molarhydrate caused a 68% and 85% inhibition respectively on i.v. administration. When tested orally in this assay the two compounds of the invention were active. Results obtained with representative compounds of the present invention in this assay are summarized hereafter in Table II.

### (c) Rat Skin Permeability. In Vivo

In this model the ability of LTE₄ to increase vascular permeability in rat skin was utilized. Anesthetized rats, pretreated for 30 minutes with an antihistamine pyrilamine maleate, 50 mg/kg i.p. and a serotonin antagonist, methyl- sergide maleate (4 mg/kg i.p.), were injected intradermally with a standard dose of LTE₄ which gave a maximum wheal (in 0.05 ml saline). After introduction of test drug (at 10 mg/kg i.v.) the animals were immediately treated intravenously with Evans blue dye i.v. (0.5%) in the tail vein resulting in the formation of a skin wheal. Thirty minutes later the animals were sacrificed and the skin wheal size was measured. The average response in 5 animals (4 intradermal injections per animal) treated with test compound was compared to that obtained in a similar group of control animals to determine the percent inhibition by the drug.

In this test representative compounds of the present invention, that is, 7-(l-methylethyl)-5-oxo-N-[4-(3-pyridinyl)butyl] -5H-thiazolo[2,3-b]quinazoline-2-carboxamide and N-[4-(1H-imidazol-1-yl)butyl]-7-(1-methylethyl)-5-oxo -SH-thiazolo[2,3-b]quinazoline-2-carboxamide dihydrochloride 0.45 molar hydrate, tested at 10 mg/kg i.v. caused a 35% and a 61% reduction, respectively, in the skin wheal size. The standard compound, 7-[3-(4-acetyl-3-hydroxy-2-propylphenoxy) -2-hydroxypropoxy]-4-oxo-8-propyl-4H-l-benzopyran-2-carboxylic acid, caused an 88% reduction in the wheal size at the same dosage. When tested orally in this assay, the representative compounds of the invention were also active while the standard compound was inactive. Results obtained with representative compounds of the present invention in this assay are summarized hereafter in Table II.

### (d) Thromboxane Synthase Inhibition, In Vitro

TXA₂ synthase activity is measured by following the conversion of ¹⁴C-prostaglandin endoperoxide (PGH₂) to ¹⁴_{C}-thromboxane A2 (TXA₂) using microsomal fractions from human platelets as enzyme source. In the aqueous incubation medium, the TXA₂ decomposes rapidly into TXB₂. The amount of TXA₂ synthase is adjusted so that under the conditions of the assay approximately 80-90% of the substrate, PGH2, is converted to product in control tubes. To prepare ¹⁴C-PGH₂, ¹⁴C-AA (50-60 mCi/mmole: Rose Chem.) is incubated with sheep seminal vesicular gland microsomes for 1.5 minutes at 37°C and then the ¹⁴C-PGH₂ is extracted with diethylether, purified on columns of Sephadex LH-20 or silicic acid, and stored in acetone at -70°C. Incubations are done as follows. Sufficient ¹⁴C-PGH₂ to yield a final substrate concentration of 10 µM (30,000 cpm) is added to the incubation tubes and then the acetone is removed under nitrogen. The tubes are placed in an ice bath and then 215 1 of ice cold phosphate buffered saline, 10 1 of ethanol (control) or of test drug in ethanol, and 25 1 of the microsomal suspension are added with mixing in that order as rapidly as possible. The tubes are incubated at 22°C for 2 minutes, the reaction is stopped and then the radioactive products and the uncoverted PGH₂ are extracted and analyzed by thin layer chromatography. The amount of ¹4 _{C}-_{PG}H converted to products is used as analyzed by thin layer chromatography. The amount of ¹⁴C-PGH₂ converted to products was used as a measure of TXA₂ synthase activity. Inhibitors were tested initially at a final concentration of 100 µM. Results obtained with representative compounds of the present invention in this assay are summarized hereafter in Table I.

### (e) Passive Cutaneous Anaphylaxis (PCA) Assav

Male Sprague-Dawley rats obtained from Charles River Breeding Laboratories were used in the PCA assay. Two methods were used to prepare serum I_{g}E antibody which is specific for egg albumin. In the first method homocytotropic antibody was obtained by injecting 180 to 200 g rats with 1.0 mg of egg albumin (Difco Laboratories, Detroit, Michigan) intramuscularly and 6.4 x 10¹⁰ organisms of Bordetella pertussis vaccine (Eli Lilly and Co., Indiana- polis, Ind.) intraperitoneally. Serum collected from each animal 18-20 days after sensitization was pooled, separated into 1 ml aliquots, and kept frozen until further use.

In the second method, rats weighing 150-200 g were immunized by the intraperitoneal injection of 0.5 ml of Bordetella pertussis vaccine (Cannaught Laboratories, Willowdale, Toronto, Canada, 20 optical units/ml) and 100 µg of egg albumin (Nutritional Biochemicals Corp., Cleveland. Ohio). Sixteen days later, Nipostrongylus brasi- liensis (3000 larvae/0.1 ml) was administered subcutaneously, and on the 21st day, 10 µg of egg albumin in 0.5 ml of normal saline was administered intraperitoneally. On the 30th day, blood samples were collected by heart puncture; the serum was separated by centrifugation and refrigerated overnight at 5°C. After a 24 hour period, the samples were assayed for antibody activity by the passive cutaneous anaphylaxis method. Those serum samples which produced an average wheal diameter of 3 mm or greater following an intradermal injection of 0.05 ml of a 1:50 dilution of serum in normal saline were pooled, divided into aliquots, and kept frozen until further use. The ability of the antiserum to produce a PCA reaction was shown to persist for 72 hours and to be inactivated by heating at 56°C for 4 hours.

The passive cutaneous anaphylaxis assay was carried out in rats weighing 190-220 g in a manner similar to that of Goose and Blair (J. Goose and A.M.J.N. Blair, Immunology, 16, 749 [1969]). Rat IgE antiovalbumin in serum was prepared as described above. The IgE antiserum was titered with normal saline so that injections of 0.05 ml produced skin wheal diameters which averaged 7 to 10 mm. After a 24 hour sensitization period, 1 ml of an aqueous solution containing a mixture of 8 mg of egg albumin (Nutritional Biochemical Corp.. Cleveland, Ohio) and 5 mg of Evans blue dye (Nepera Chemical Co., Inc., Harriman, N.Y.) was administered intravenously to each rat. Forty minutes after challenge, each animal was sacrificed by cervical dislocation, and the dorsal skin was removed for examination. The long and short axes of the blued area were measured on the inner surface of the skin with a metric vernier caliper (VWR Scientific Division of Univar, Rochester, N.Y.), and the average diameter was determined for each reaction. Test compounds were administered 5 min before the intravenous injection of the dye and antigen, except as indicated in Table III. Logarithmically spaced doses were administered orally at the time of peak activity to determine the dose that caused a 50% inhibition (ID₅₀) in the expected wheal size. The procedure was usually conducted on five animals in the control and each of the treatment groups. Statistical analysis of the test results was conduced by the Student's t test of control vs. various treatments. The ID₅₀ and 95% limits were determined by a modification of the Berkson minimum logic chi square method (J. Berkson, J. Am. Stat. Assoc., 48, 565 [1953]).

### (f) In Vivo Screening Procedure for SRS-A Synthesis Inhibitors in Guinea Pigs

Paralyzed, artificially ventilated, actively sensitized guinea pigs are pretreated with indomethacin (to divert the flow of endogenous arachidonic acid through the lipoxygenase pathway), pyrilamine maleate (to block any bronchoconstriction due to released histamine) and propranol (to enhance the sensitivity of the guinea pig bronchoconstriction to SRS-A) prior to challenging the animal with antigen. Under these conditions the resulting antigen-induced bronchoconstriction has been characterized as being attributable to the synthesis and release of SRS-A in the guinea pig lung. Test compounds are initially studies for their inhibitory properties in this test system by the intravenous route (at 10 mg/kg) given 1 minute prior to antigen challenge. In this test, 7-(l-methylethyl)-5-oxo-N-[4-(2-pyridinyl)butyl] -5H-thiazolo[2.3-b]quinazoline-2-carboxamide (10 mg/kg i.v.) caused a 44% inhibition of the bronchoconstriction.

Contained in Tables I, II and III which follow are results obtained when compounds of the present invention are subjected to the above-described tests.

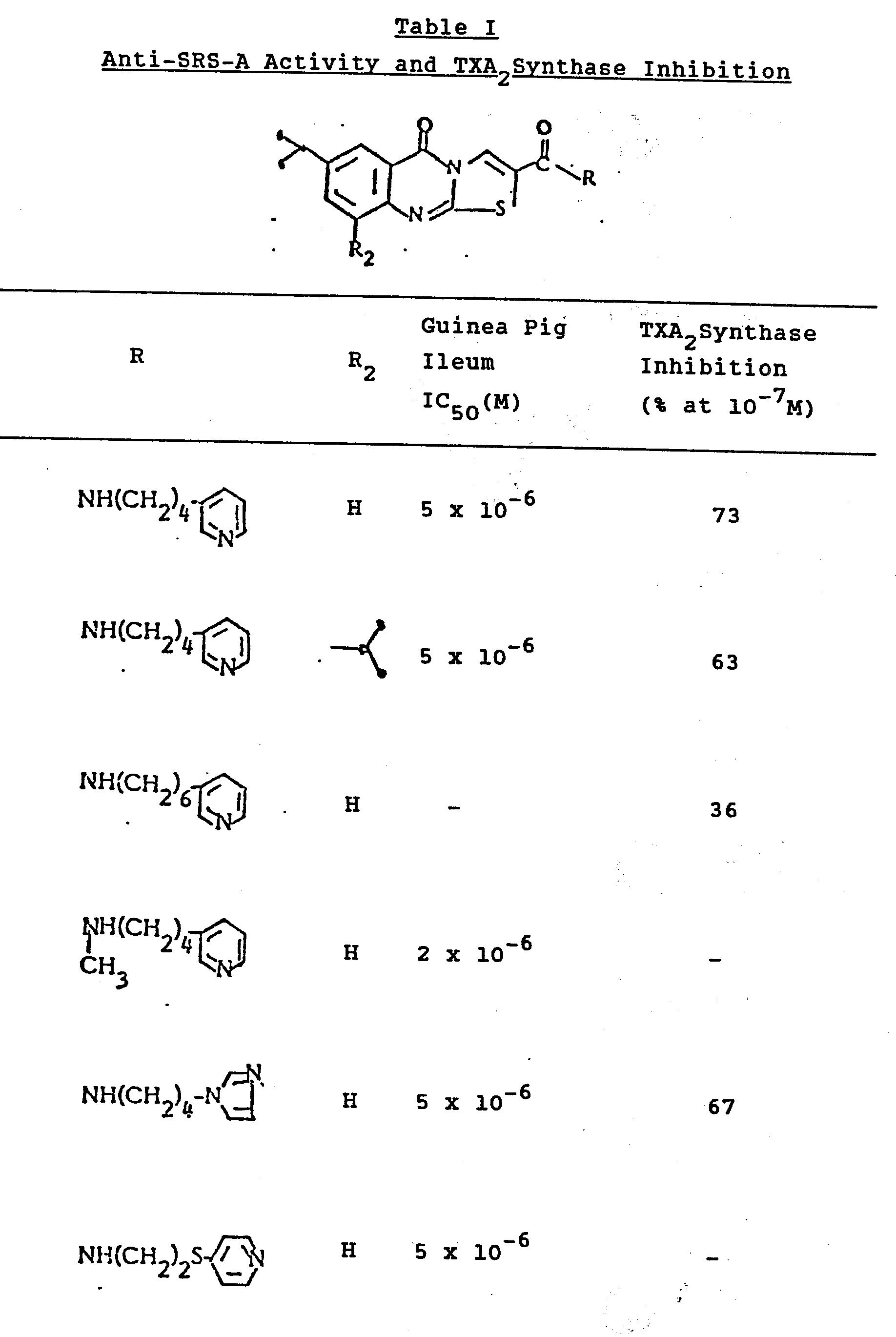

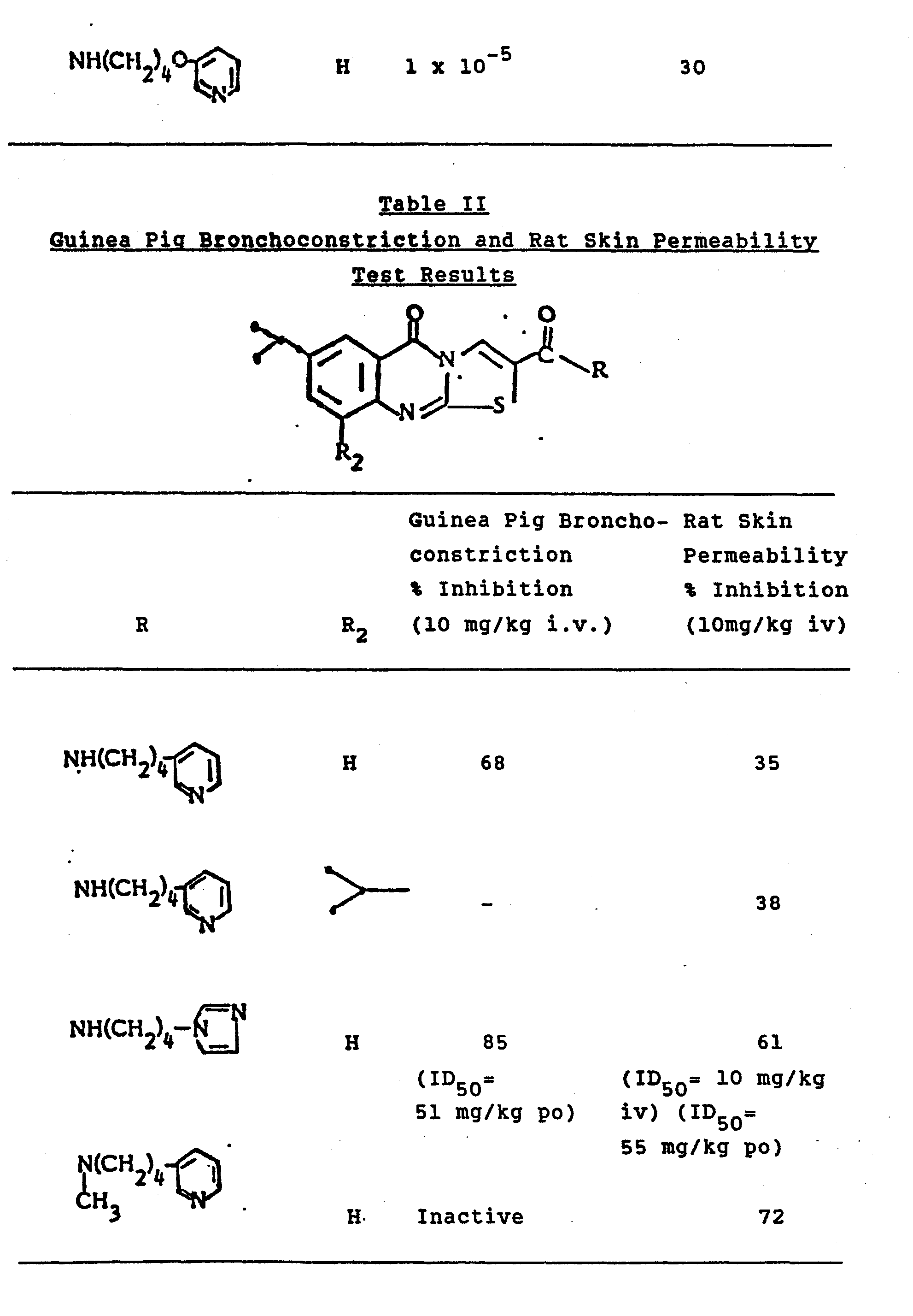

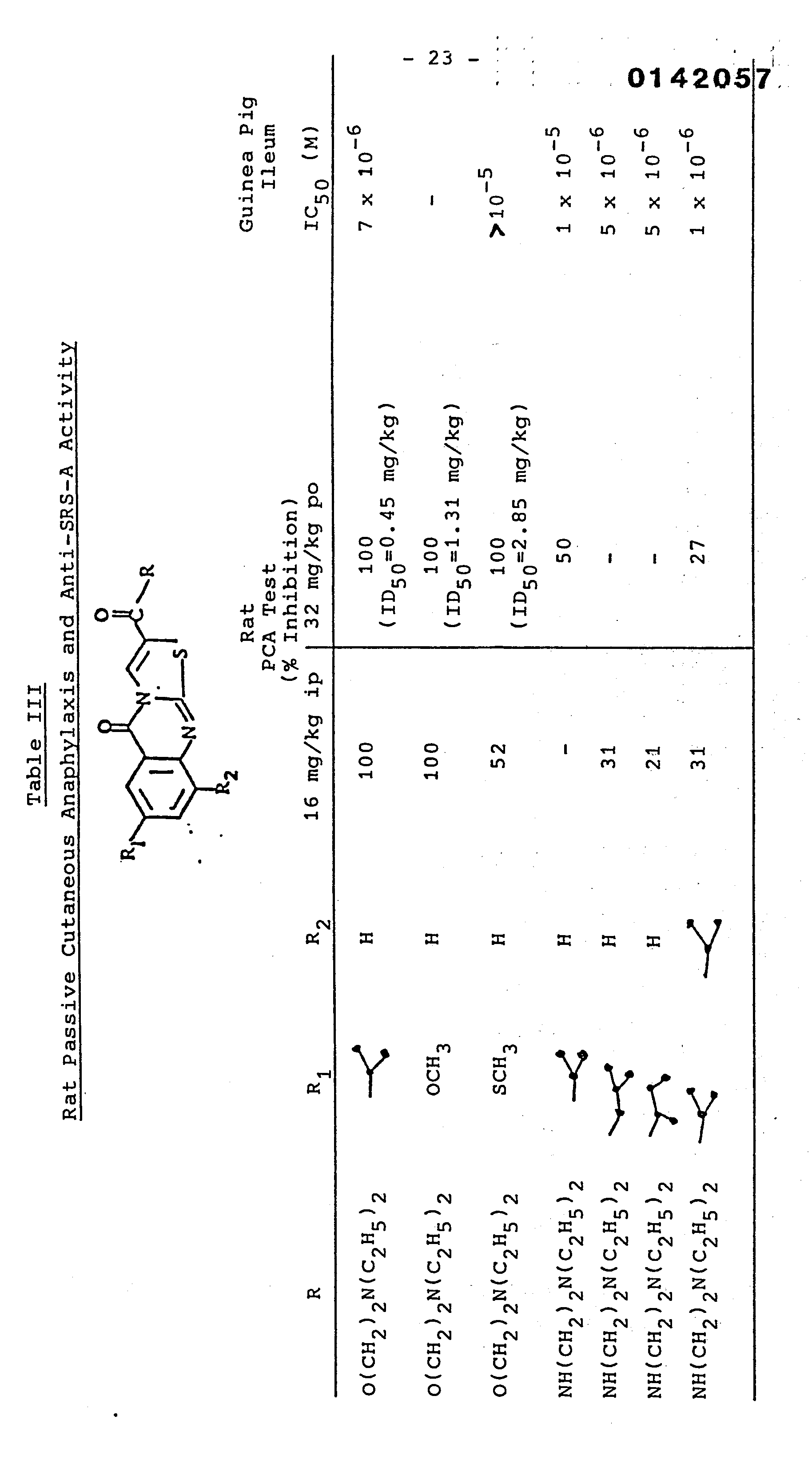

The compounds of formula I and their pharmaceutically acceptable acid addition salts can be administered orally or parenterally as anti-allergic agents, for example, in the prophylactic treatment of bronchial asthma, with dosage adjustments for individual requirements. They can be administered therapeutically, for example, orally or parenterally, by incorporating a therapeutic dosage in a conventional dosage form, such as tablets, capsules, elixirs, suspensions, solutions or the like. They can be administered in mixture with conventional pharmaceutical carriers or excipients, such as, for example, corn starch, calcium stearate, magnesium carbonate, calcium silicate, dicalcium phosphate, talc, lactose, and the like. Moreover, they can be administered in the presence of buffers, or agents used to adjust to isotonicity and the pharmaceutical dosage forms can, if desired, be subjected to conventional pharmaceutical expedients such as, for example, sterilization. As states above, the dosage can be adjusted to individual requirements. They can also contain other therapeutically valuable substances.

The frequency with which any such dosage form will be administered to a mammal will vary, depending upon the quantity of active medicament present therein and the needs and requirements of the mammal. Dosages of a compound of formula I and its pharmaceutically acceptable acid addition salts contemplated for use in practicing the invention are in the range of from about 100 to about 1500 mg per day, either as a single dose or in divided doses. It is to be understood, however, that the above description and dosage strengths and the tablet and capsule descriptions set forth herein are exemplary only and that they do not, to any extent, limit the scope or practice of this invention.

The examples which follow further illustrate the invention. All temperatures are given in degrees centigrade.

### Example 1

### 7-(1-Methylethyl)-5-oxo-5H-thiazolo[2,3-b]quinazoline-2-carboxylic acid

A solution of 35.8 g (0.2 mol) of 2-amino-5-(l-methylethyl)benzoic acid, 35.5 g (0.2 mol) of 5-carbomethoxy-2--chlorothiazole and 28 ml of formic acid in 370 ml of 2-methoxyethanol was stirred at reflux for 10 hr. On cooling a yellow solid crystallized and was filtered to give 30.3 g of methyl ester which was suspended in 1.5 1 of methanol and 500 ml of 1N NaOH. After stirring for 17 hr at 25°, most of the solvent was removed in vacuo. Water (500 ml) was added to the residue and the pH was adjusted to 1.5 with 6N HC1. The resultant yellow solid was filtered and recrystallized from acetic acid to yield 24.8 g, mp 232-234°, (43% yield) of 7-(l-methylethyl)-5-oxo -5H-thiazolo[2,3-b]quinazoline-2--carboxylic acid.

### Example 2

### 7,9-Bis(1-methylethyl)-5-oxo-5H-thiazolo[2,3-b]quinazoline-2-carboxylic acid

A solution of 5.0 g (0.023 mol) of 2-amino-3,5-bis(1--methylethyl)benzoic acid, 4.0 g (0.023 mol) of 5-carbome- thoxy-2-chlorothiazole and 3.5 ml of formic acid in 35 ml of 2-methoxyethanol was stirred at reflux for 20 hr. The solvent was removed in vacuo and the residue was triturated with ether and the resultant solid was filtered to yield 2.2 g, mp 149-155°, of methyl ester. This was suspended in 90 ml of methanol and 65 ml of 1N NaOH and stirred at 25° for 22 hr. After most of the solvent was removed in vacuo, water (100 ml) was added and the pH was adjusted to 2.0 with 6N HC1. The resultant solid was filtered and recrystallized from acetic acid and then from methanol to yield 1.9 g, mp 219-222°, (25% yield) of 7.9-bis(1-methylethyl)-5-oxo -5H-thiazolo[2.3-b]quinazoline-2-carboxylic acid.

### Example 3

### 7-(1-Methvlethyl)-5-oxo-N-(4-(3-pyridinyl)butyl-5H-thiazolo(2,3-quinazoline-2-carboxamide

To a stirred suspension of 2.00 g (0.007 mol) of 7-(l-methylethyl)-5-oxo -5H-thiazolo[2,3-b]quinazoline-2--carboxylic acid and 1.16 g (0.0077 mol) of 3-pyridine butanamine in 20 ml of anhydrous dimethylformamide cooled at -10° was added dropwise 1.66 ml (0.0077 mol) of diphenylphosphoryl azide followed by the dropwise addition of 2.1 ml (0.0147 mol) of triethylamine. Anhydrous dimethylformamide (20 ml) was added and stirring at -5° was continued for 2.5 hr. The cooling bath was removed and stirring was continued at 25° for 2.5 hr. The dimethylformamide was removed in vacuo, CH₁Cl₂ (100 ml) was added to the residue and the solution was washed with 5% NaHCO₃ solution. The dried (MgSO₄) extract was concentrated in vacuo to a yellow solid which was chromatographed on 200 g of silica gel. Elution with a solvent mixture of CH₂Cl₂ (190): 95% CH₃0H (10): conc. NH₄0H (0.1) gave 1.09 g of light yellow solid which was crystallized from CH₂Cl₂ - Et₂O to yield 0.95 g (33% yield), mp 173-176°, of 7-(1-methylethyl)-5-oxo--N-[4-(3-pyridinyl)butyl] -5H-thiazolo[2,3-b]quinazoline-2--carboxamide.

### Example 4

### 7,9-Bis(1-methylethyl)-5-oxo-N-(4-(3-pyridinyl)butyll-5H-thiazolo[2,3-blquinazoline-2-carboxamide hydrochloride

To a stirred suspension of 1.98 g (0.006 mol) of 7.9-bis(l-methylethyl)-5-oxo -5H-thiazolo[2,3-b]quinazoline-2-carboxylic acid and 1.08 g (0.0072 mol) of 3-pyridine butanamine in 100 ml of anhydrous dimethylformamide at -5° was added dropwise 1.6 ml (0.0072 mol) of diphenylphosphoryl azide followed by the dropwise addition of 1.8 ml (0.0126 mol) of triethylamine. Stirring was continued at -5° for 2.5 hr and then at 25° for 2 hr. The dimethylformamide was removed in vacuo, ethyl acetate was added to the residue and the solution was washed with 5% NaHCO₃ solution and with water. After drying (MgSO₄), the extract was concentrated in vacuo to yield a yellow solid. Trituration with ether gave 1.33 g which was dissolved in 50 ml of CH₂C1₂ and converted to the hydrochloride salt by the addition of 1.3 ml (0.0086 mol) of 6.5N HC1 in methanol. Ether (100 ml) was added with stirring and the resultant solid was filtered and recrystallized from 2-propanol-methanol to yield 1.11 g (37% yield), mp 230-233°, of 7,9-bis(1-methylethyl)-5-oxo-N--[4-(3-pyridinyl)butyl] -5H-thiazolo[2,3-b]quinazoline-2--carboxamide hydrochloride.

### Example 5

### N-Methyl-7-(1-methylethyl)-5-oxo-N-[4-(3-pyridinyl)butyl]-5H-thiazolo[2,3-b]quinazoline-2-carboxamide

A suspension of 1.20 g (0.0042 mol) of 7-(l-methylethyl)-5-oxo -5H-thiazolo[2,3-b]quinazoline-2-carboxylic acid in 20 ml of thionyl chloride was stirred at reflux for 3 hr. The excess thionyl chloride was removed in vacuo to yield a yellow solid acid chloride which was dissolved in 20 ml of refluxing anhydrous toluene and 1.00 g (0.0063 mol) of N-methyl-3-pyridine butanamine in 10 ml of anhydrous toluene was added dropwise with stirring over 20 min. The reaction mixture was then stirred at reflux for 5 hr. The solvent was removed in vacuo, CH₂C1₂ and saturated NaHCO₃ solution were added to the residue. The organic layer was dried (MgS0₄) and concentrated in vacuo to an oil which was chromatographed on 200 g of silica gel. Elution with a solvent of CH₂C1₂ (190): 95% CH₃OH (10): conc. NH₄0H (0.1) gave 1.2 g of an oil which was crystallized from CH₂Cl₂-ether-hexane to yield 0.91 g (50% yield), mp 86-89°, of N-methyl-7-(l-methylethyl)-5-oxo-N-[4--(3-pyridinyl)butyl] -5H-thiazolo[2,3-b]quinazoline-2-carboxamide.

### Example 6

### N-[4-(1H-Imidazol-1-yl)butyl]-7-(1-methylethyl)-5-oxo-5H-thiazolo[2,3-b]quinazoline-2-carboxamide dihydrochloride 0.45 molar hydrate

A suspension of 2.40 g (0.0083 mol) of 7-(1-methylethyl)-5-oxo -5H-thiazolo[2,3-b]quinazoline-2-carboxylic acid in 40 ml of thionyl chloride was stirred at reflux for 3 hr. The excess thionyl chloride was removed in vacuo and the residue was dissolved in refluxing anhydrous toluene and 1.75 g (0.0125 mol) of (1H-imidazal-1-yl)-1-butanamine in 20 ml of anhydrous toluene was added dropwise over 30 min with stirring. The reaction mixture was then stirred at reflux for 5 hr. After cooling, the resultant solid was filtered and suspended in 100 ml of saturated NaHCO₃ solution and extracted with CH₂Cl₂. The dried (Na₂S0₄) extract was converted to the hydrochloride salt by the addition of 3.0 ml (0.18 mol) of 6N HC1 in methanol. Ether (100 ml) was added and the resultant solid was filtered and recrystallized from 2-propanol-methanol to yield 2.02 g (54% yield), mp 204-207°, of N-[4-(1H-imidazol-1-yl)butyl]-7-(1--methylethyl)-5-oxo -5H-thiazolo[2.3-b]quinazoline-2-carboxamide dihydrochloride 0.45 molar hydrate.

### Example 7

### 7-(1-Methylethyl)-5-oxo-N-[2-(4-pyridinylthio)ethyl]-5H-thiazolo[2,3-blauinazoline-2-carboxamide

The acid chloride was prepared from 2.4 g (0.0083 mol) of 7-(l-methylethyl)-5-oxo -5H-thiazolo[2,3-b]quinazoline-2--carboxylic acid using thionyl chloride. To the acid chloride in 150 ml of anhydrous toluene stirred at reflux was added dropwise 1.92 g (0.0125 mol) of 2-(4-pyridinylthio)-ethanamine in 10 ml of anhydrous toluene. The reaction mixture was then stirred at reflux for 5 hr. After cooling, the solid was filtered and recrystallized from methanol to give 2.65 g of yellow solid. This hydrochloride salt was stirred with 150 ml of saturated NaHCO₃ solution and 200 ml of CH₂Cl₂. The CH₂Cl₂ layer was dried (MgSO₄) and concentrated in vacuo to a solid which was recrystallized from methanol to give 2.16 g (51% yield), mp 188-192⁰, of 7-(l-methylethyl)-5-oxo-N-[2-(4-pyridinylthio)ethyl] -5H-thiazolo[2,3-b]quinazoline-2-carboxamide.

### Example 8

### 7-(1-Methylethyl)-5-oxo-N-[4-(3-pyridinyloxy)butyl]-5H-thiazolo[2,3-b]quinazoline-2-carboxamide hydrochloride

The acid chloride was prepared from 2.4 g (0.0083 mol) of 7-(1-methylethyl)-5-oxo -5H-thiazolo[2,3-b]quinazoline-2--carboxylic acid using thionyl chloride. To the acid chloride in 50 ml of anhydrous toluene stirred at reflux was added dropwise 2.1 g (0.0125 mol) of 4-(3-pyridinyloxy)-l--butanamine in 20 ml of anhydrous toluene over 30 min. The reaction mixture was stirred at reflux for 2.5 hr. After cooling, the solid was filtered, suspended in 150 ml of saturated NaHC0₃ solution and extracted with CH₂Cl₂. The extract was dried (Na₂SO₄) and converted to the hydrochloride salt by the addition of 4.2 ml (0.025 mol) of 6N HCl in methanol. Addition of ether gave a precipitate which was filtered and recrystallized from 2-propanol-methanol to yield 3.46 g (88% yield), mp 206-209°, of 7-(1--methylethyl)-5-oxo-N-[4-(3-pyridinyloxy)butyl] -5H-thiazo- lo[2,3-b]quinazoline-2-carboxamide hydrochloride.

The 4-(3-pyridinyloxy)-l-butanamine used as a starting material above can be prepared as follows:

### Example 9

### 4-(3-Pvridinyloxv)butyronitrile

Sodium hydride (13.3 g, 0.32 mol, 57% oil dispersion) was washed with pentane to remove the oil and 26.1 g (0.275 mol) of 3-hydroxypyridine in 300 ml of anhydrous dimethylformamide was added with cooling in a cold water bath. The mixture was then stirred and heated at 60° for 1 hour. After cooling to 25°, 40.7 g (0.275 mol) of 4-bromobutyronitrile in 40 ml of anhydrous dimethylformamide was added dropwise over 1 hour with cooling to keep the reaction temperature below 30°. The reaction mixture was then stirred at 25° for 17 hours and the solvent was removed in vacuo. The residue was treated with 100 ml of water and the product was extracted with CH₂C1₂. After washing the extract with 1N NaOH and then with H₂0, it was dried (MgSO ) and concentrated in vacuo to an oil which was distilled to yield 25.1 g, b.p. 133-135°/53 Pa, (56% yield), of 4-(3-pyridinyloxy)butyronitrile.

### 4-(3-Pyridinyloxy)-1-butanamine

To 2.9 g (0.073 mol) of lithium aluminum hydride stirred at 25° in 100 ml of anhydrous ether at 25° was added dropwise 4.7 g (0.029 mol) of 4-(3-pyridinylox)butyronitrile in 30 ml of ether and 20 ml of anhydrous tetrahydrofuran over 15 minutes. The reaction mixture was then stirred at reflux for 3.5 hours, cooled in an ice bath during the addition of 3 ml of H₂0, followed by 3 ml of 15% NaOH solution and finally 9 ml of H₂0. The granular solid was filtered, washed with CHC1₃ and the filtrate was concentrated in vacuo. The residue was dissolved in CHCl₃. washed with H₂0, dried (MgSO₄) and concentrated in vacuo to an oil. Distillation gave 2.6 g, b.p. 103-107°/67 Pa (53% yield) of 4-(3--pyridinyloxy)-l-butanamine which was analyzed as the dihydrochloride obtained from 2-propanol-ether, m.p. 127-131°.

### Example 10

### 7-(1-Methylethyl)-5-oxo-N-(6-(3-pyridinyl)hexyl]-5H-thiazolo-(2,3-b]quinazoline-2-carboxamide

A mixture of 7-(l-methylethyl)-5-oxo -5H-thiazolo-[2,3-b]quinazoline-2-carboxylic acid and 40 ml of thionyl chloride was stirred at reflux for 3 hr. The excess thionyl chloride was removed in vacuo to yield the acid chloride as a solid which was dissolved in 70 ml of refluxing anhydrous toluene. This solution was stirred at reflux during the dropwise addition of 2.23 g (0.013 mol) of 3-pyridine hexanamine in 20 ml of anhydrous toluene over 15 minutes. The mixture was stirred at reflux for 5 hr and then the solvent was removed. The residue was dissolved in CH₂Cl₂, washed with NaHCO₃ solution, washed with water, dried (Na₂SO₄) and the CH₂C1₂ was removed in vacuo. The solid residue was crystallized from ethyl acetate once and from isopropyl alcohol once to yield 2.7 g (66%), m.p. 171-173°, of 7-(l-methylethyl)-5₋oxo-N-[6-(3-pyridinyl)-hexyl] -5H-thiazolo[2,3-b]quinazoline-2-carboxamide.

### Example 11

### 7-(1-Methylethyl)-5-oxo-N-[4-(2-pyridinyl)butyl -5H-thiazo- lo[2,3-blquinazoline-2-carboxamide

A mixture of 2 g (0.007 mol) of 7-(l-methylethyl)-5-oxo--5H-thiazolo[2,3-b]quinazoline -2-carboxylic acid and 25 ml of thionyl chloride was stirred at reflux for 2 hours. The excess thionyl chloride was removed in vacuo to give the acid chloride as a solid which was dissolved in 100 ml of anhydrous dimethylformamide. To this solution stirred at 0° was added 1.26 g (0.0084 mol) of 4-[2-pyridinyl)butanamine dissolved in 20 ml of dimethylformamide. After stirring at room temperature for 17 hours the solvent was removed in vacuo and saturated NaHCO₃ was added to the residue. The product was extracted with methylene chloride. The dried (MgS0₄) extract was concentrated in vacuo to a solid which was crystallized from ethyl acetate to yield 1.2 g, m.p. 177-179⁰, (41% yield) of 7-(1-methylethyl) -5-oxo-N-[4-(2--pyridinyl)butyl]-5H-thiazolo[2,3-b]quinazoline -2-carboxamide.

### Example 12

### N-(2-(Diethylamino)ethyl]-7-(1-methylethyl) -5-oxo-5H-thia- zolo(2,3-blquinazoline -2-carboxamide hydrochloride

A suspension of 1.15 g (0.004 ml) of 7-(l-methylethyl)--5-oxo-5H-thiazolo[2,3-b]quinazoline -2-carboxylic acid in 20 ml of thionyl chloride was stirred at reflux for 3 hours. The clear solution was concentrated in vacuo to a yellow solid. Anhydrous toluene was added and concentrated in vacuo in order to remove residual thionyl chloride. The residue was dissolved in 20 ml of refluxing anhydrous toluene and stirred while a solution of 0.70 g (0.006 mole) of N,N-di- ethylethylenediamine in 5 ml of anhydrous toluene was added dropwise over 20 min. The reaction mixture was stirred at reflux for 4 hours. The solvent was removed in vacuo, the residue was treated with excess saturated sodium bicarbonate solution and the product was extracted with methylene chlo₋ ride. The dried (MgSO₄) extract was concentrated in vacuo to a solid which was stirred with hexane and filtered to yield a tan solid (1.13 g). This solid was dissolved in 25 ml of CH₂Cl₂ and 0.8 ml of 4.0 M HC1 in methanol was added. Addition of 100 ml of hexane caused precipitation of the pure product which was filtered to give 1.18 g (70% yield), mp 228-231⁰, of N-[2-diethylamino)ethyl]-7-(l--methylethyl) -5-oxo-5H-thiazolo[2,3-b]quinazoline -2-carboxamide hydrochloride.

### Example 13

### 7,9-Bis(1-methylethyl)-N-[2-(diethylamino)ethyl] -5-oxo-5H--thiazolo[2,3-b]quinazoline -2-carboxamide hydrochloride

2.00 g (0.0061 mole) of 7,9-bis(1-methylethyl)-5-oxo-5H--thiazolo[2.3-b]quinazoline -2-carboxylic acid was converted to the acid chloride by the usual procedure. The acid chloride was dissolved in 35 ml of anhydrous toluene at reflux and stirred while a solution of 1.05 g (0.0091 mole) of N,N-diethylethylenediamine in 10 ml of anhydrous toluene was added dropwise over 45 min. The reaction mixture was stirred at reflux for 4 hours and worked up as usual to give 1.88 g of a tan solid which was dissolved in 10 ml of CH₂C1₂ and treated with 1.3 ml (0.0048 mole) of 3.8 M HC1 in methanol. Addition of 100 ml of hexane caused precipitation of the product which was filtered to give, after recrystallization from CH₂Cl₂-Et₂O, 1.80 g (64% yield), mp 247-249°, of 7,9-bis(1-methylethyl)-N-[2-(diethylamino)-ethyl] -5-oxo-5H-thiazolo[2,3-b]quinazoline -2-carboxamide hydrochloride.

### Example 14

### N-[2-(Diethylamino)ethyl]-7-(2-methylpropyl) -5-oxo-5H-thia- zolo(2,3-b]quinazoline -2-carboxamide hydrochloride

2.21 g (0.0073 mole) of 7-(2-methylpropyl)-5-oxo-5H--thiazolo[2,3-b]quinazoline -2-carboxylic acid was converted to the acid chloride in the usual manner. The acid chloride was dissolved in 35 ml of refluxing anhydrous toluene and stirred while a solution of 1.27 g (0.011 mole) of N,N-di- ethylethylenediamine in 10 ml of anhydrous toluene was added dropwise over 50 min. The reaction mixture was stirred at reflux for 4 hours and worked up as usual to give 2.48 g of a tan solid which was dissolved in 30 ml of CH₂Cl₂ and treated with 1.85 ml (0.0068 mole) of 3.7 M HC1 in methanol. Addition of 100 ml of ether caused precipitation of the product which was filtered and recrystallized from CH₂C1₂--Et₂0 to give 2.38 g (75% yield), mp 214-217⁰, of N-[2--(diethylamino)ethyl] -7-(2-methylpropyl)-5-oxo-5H-thiazo- lo[2,3-b]quinazoline -2-carboxamide hydrochloride.

### Example 15

### N-[2-(Diethylamino)ethyl]-7-(1-methylpropyl) -5-oxo-5H-thia- zolo[2,3-b]^{q}uinazoline-2-carboxamide

2.5 g (0.0083 mole) of 7-(1-methylpropyl) -5-oxo-5H--thiazolo[2,3-b]quinazoline-2-carboxylic acid was converted to the acid chloride as usual. The acid chloride was dissolved in 35 ml of anhydrous toluene at reflux and stirred while a solution of 1.45 g (0.0125 mole) of N,N-diethyl- ethylenediamine in 10 ml of anhydrous toluene was added dropwise over 30 min. The reaction mixture was stirred at reflux for 4 hours and worked up as usual to give 2.62 g of a tan solid which was recrystallized twice from CH₂Cl₂--hexane to give 1.28 g (39% yield), mp 100-101°, of N-[2--(diethylamino)ethyl] -7-(l-methylpropyl)-5-oxo-5H-thiazo- lo[2,3-b]quinazoline -2-carboxamide.

### Example 16

### 7-(1-Methylethyl)-5-oxo-5H-thiazolo[2,3-b]quinazoline -2-carboxylic acid(2-diethylaminoethyl) ester hydrochloride

A mixture of 0.713 g (2.48 mmol) of 7-(1-methylethyl)-5--oxo-5H-thiazolo[2,3-b]quinazoline -2-carboxylic acid and 10 ml of thionyl chloride was stirred and refluxed for 3.5 hours. The excess thionyl chloride was removed in vacuo to give a yellow solid. Anhydrous toluene was added and removed in vacuo several times to remove traces of residual thionyl chloride. The resultant solid was dissolved in 10 ml of anhydrous toluene and 0.36 ml (2.73 mmol) of N,N-diethyl- amino ethanol was added and stirred at room temperature for 4 days. The solvent was removed in vacuo, 25 ml of saturated sodium bicarbonate solution was added and the product was extracted with methylene chloride. The dried (MgSO₄) extract was treated with 1.43 ml (2.5 mmol) of 1.81 M HC1 in methanol and then with 200 ml of ether. The yellow solid was filtered and recrystallized twice from methylene chloride--ether to yield 0.560 g, mp 188-189°, of 7-(1-methylethyl)--5-oxo-5H-thiazolo[2,3-b]quinazoline -2-carboxylic acid (2-diethylaminoethyl) ester hydrochloride.

### Example 17

### 7-(Methylthio)-5-oxo-5H-thiazolo[2,3-b]quinazoline -2-carboxylic acid (2-diethylaminoethyl) ester hydrochloride

A mixture of 0.750 g (2.57 mmol) of 7-(methylthio)-5--oxo-5H-thiazolo[2,3-b]quinazoline -2-carboxylic acid and 15 ml of thionyl chloride was refluxed and stirred for 4 hours. The excess thionyl chloride was removed in vacuo to give a solid. Anhydrous toluene was added and removed in vacuo several times to remove traces of residual thionyl chloride. The residue was dissolved in 65 ml of toluene and 0.38 ml (2.83-mmol) of N.N-diethylamino ethanol was added and stirred at room temperature for 2 days. The solvent was removed in vacuo, 50 ml of saturated sodium bicarbonate solution was added and the product was extracted with methylene chloride. The dried (MgSO₄) extract was treated with 1.7 ml (2.38 mmol) of 1.64 M HC1 in methanol and then with 250 ml of ether. The solid was filtered and recrystallized from methylene chloride-ether to yield 0.555 g, mp 215-216⁰, of pure 7-(methylthio)-5-oxo-5H-thiazolo[2.3-b]quinazoline -2-carboxylic acid (2-diethylaminoethyl) ester hydrochloride.

### Example 18

### 3-Pyridinehexanamine

A solution of 52.15 g of 3-pyridinehexanenitrile in 600 ml of methanol and 13 ml of triethylamine was hydrogenated over 13 g of Raney cobalt at an initial hydrogen pressure of 6,9·10⁶ Pa and 100°. The cooled mixture was filtered and concentrated. The residue was distilled to give 46.5 g (87%) of 3-pyridinehexanamine. bp 102-10₇⁰/27 Pa. This material was purified through its phthalimide which was formed by reaction with 39.53 g of phthalic anhydride in 300 ml of glacial acetic acid at reflux overnight. The residue obtained after evaporation of the solvent was dissolved in 300 ml of ethyl acetate, washed with dilute sodium hydroxide and sodium bicarbonate, dried over potassium carbonate and concentrated. The residue was crystallized from ethyl acetate-hexane to give 62.87 g (77%) of 2-[6-(3-pyridinyl)hexyl]-lH-isoindole-1,3(2H)-dione, mp 89-92°. A solution of this material in 880 ml of ethanol and 33 ml of hydrazine hydrate was heated to reflux for 3 hours. The cooled mixture was filtered and concentrated. The residue was taken up in 500 ml of dichloromethane and was washed with 10% sodium hydroxide and dried over potassium carbonate. The residue obtained after concentration was distilled to give 31.6 g (60% based on starting nitrile) of 3-pyridinehexanamine, bp 140-150⁰/40 Pa which gave a single peak on gas chromatography.

### Example 19

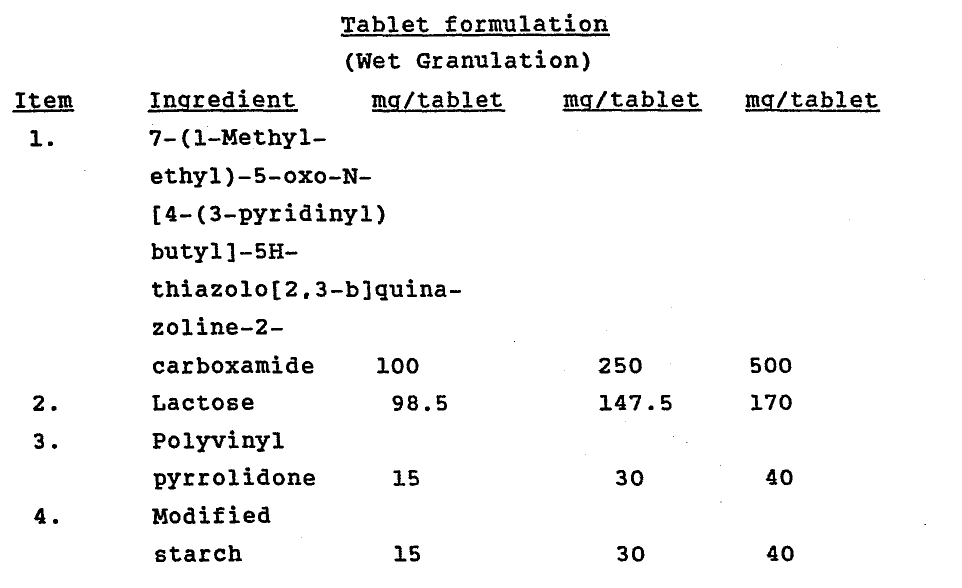

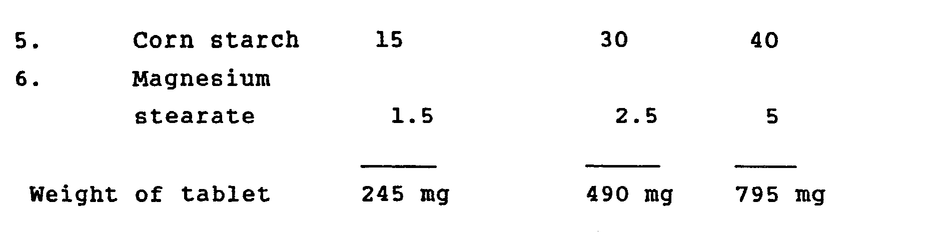

### Procedure:

1) Mix items 1, 2, 4 and 5 in a suitable mixer, granulate with polyvinyl pyrrolidone and dissolve in water/alco- hol. Dry the granulation. Mill the dry granulation through a suitable mill.
2) Add magnesium stearate and compress on a suitable press.

### Example 20

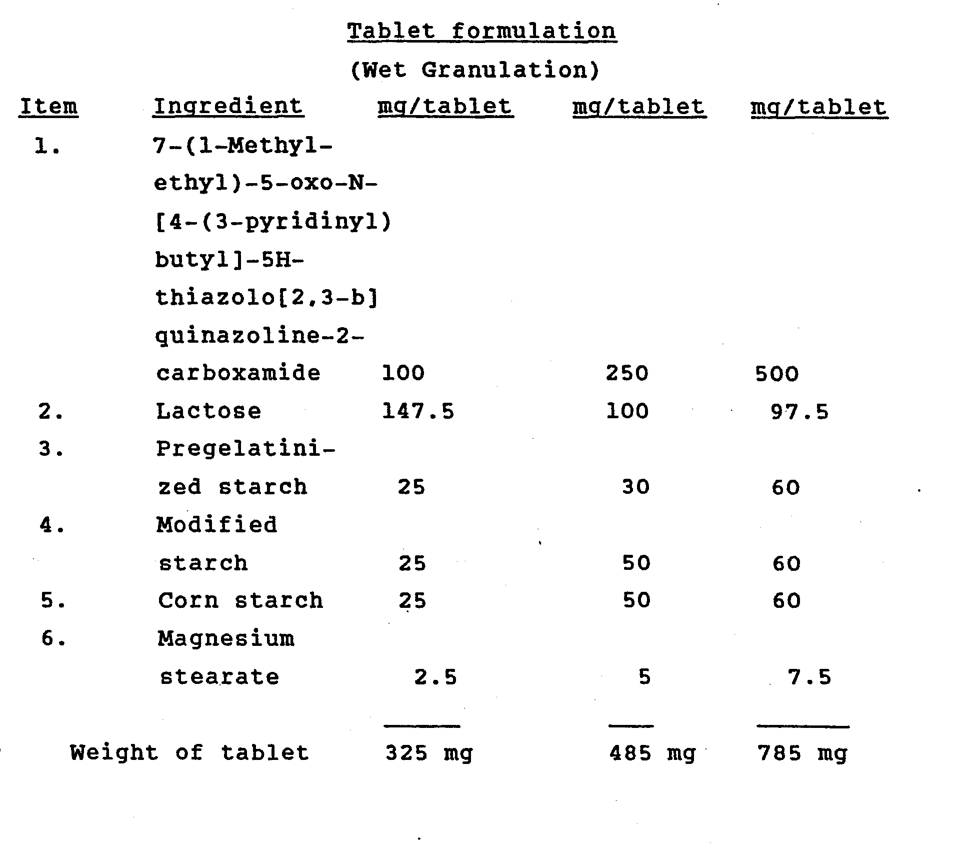

### Procedure:

1) Mix items 1, 2, 3, 4 and 5 in a suitable mixer, granulate with water, and dry over night in a suitable oven. Mill through suitable mill.
2) Mix with item 6 and compress on a suitable press.

### Example 21

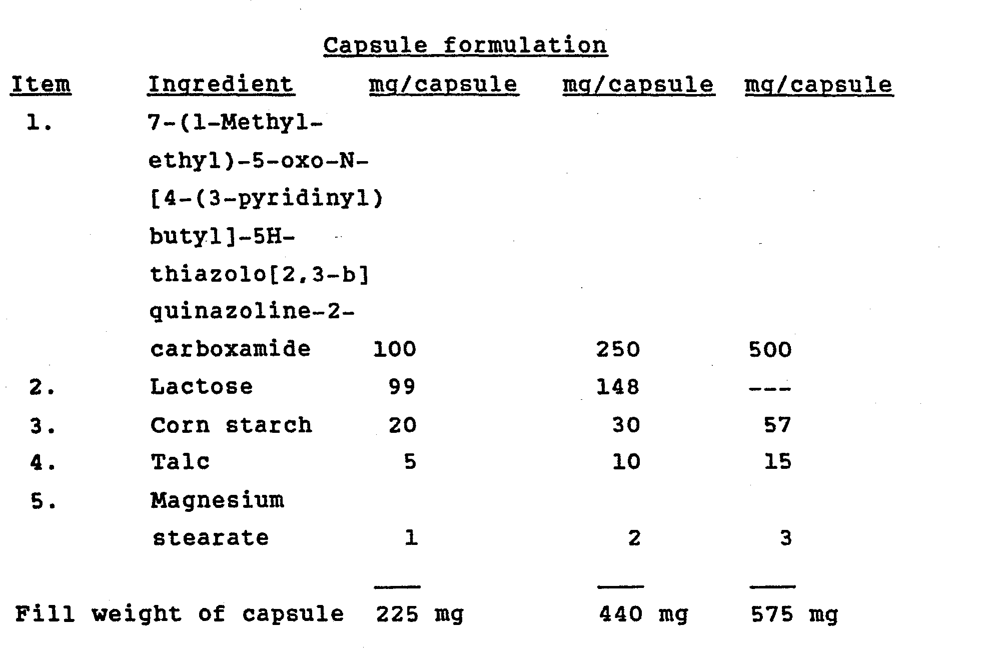

### Procedure:

1) Mix items 1, 2 and 3 in a suitable mixer. Mill through suitable mill.
2) Mix the mixture in Step 1 with items 4 and 5 and fill on a suitable machine.

## Claims

1. A thiazolo[2,3-b]quinazoline of the general formula 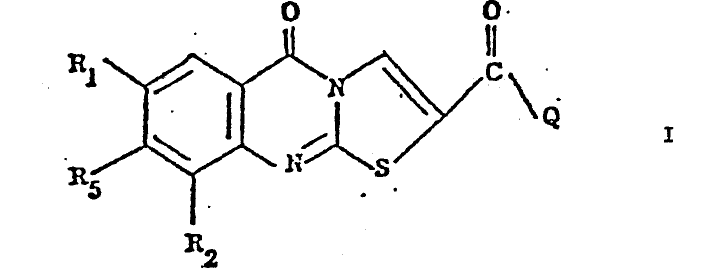 wherein R₁ is hydrogen, lower alkyl, lower cycloalkyl, lower alkoxy, hydroxy, halogen, lower alkylthio, lower alkylsulfinyl, lower alkylsulfonyl, di-lower alkyl--N(CH₂)ₙO- or 2-hydroxyethoxy; n is 2 to 7; R2is hydrogen, lower alkyl or lower alkoxy; R₅ is hydrogen or lower alkyl: provided that if R₂ is other than hydrogen, R₅ is hydrogen; Q is one of the groups 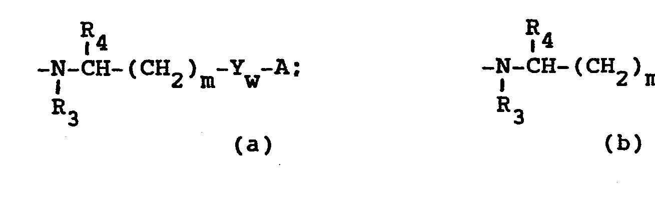 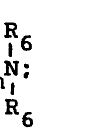 and 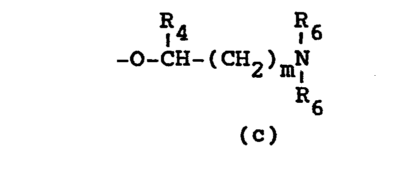 wherein R₃ is hydrogen or methyl: R₄ is hydrogen or lower alkyl: R₆ is lower alkyl: m is 1 to 7: w is zero or one: Y is -NH-: -NH-CH₂-; -O-; or -S.: and A is an unsubstituted or substituted heterocyclic radical; or a pharmaceutically acceptable acid addition salt thereof.

2. A compound in accordance with claim 1, wherein A is selected from the group consisting of pyridinyl, pyrimidinyl, imidazolyl, furyl, thiazolyl, oxazolyl, pyrazinyl, quinolinyl, isoquinolinyl, quinazolinyl, 1,2,4-triazinyl. benzimidazolyl and pyridazinyl.

3. A compound in accordance with claim 1 or 2, wherein R₁ is lower alkyl, preferably isopropyl, R₂ is hydrogen or lower alkyl, R₃ is hydrogen or methyl, R₄ is hydrogen, R₅ is hydrogen and A is 2-, 3- or 4-pyridinyl or 1-imidazolyl. 4. 7-(l-Methylethyl)-5-oxo-N-[4-(3-pyridinyl)butyl -5H-thiazolo[2,3-b]quinazoline-2-carboxamide or a salt thereof.

5. 7,9-Bis-(1-methylethyl) -5-oxo-N-[4-(3-pyridinyl)-butyl-5H-thiazolo[2,3-b]quinazoline -2-carboxamide or a salt thereof.

6. N-Methyl-7-(1-methylethyl) -5-oxo-N-[4-(3-pyridinyl)-butyl]-5H-thiazolo[2.3-b]quinazoline -2-carboxamide or a salt thereof.

7. 7-(1-Methylethyl)-5-oxo-N-[2-(4-pyridinylthio)ethyl] -5H-thiazolo[2,3-b]quinazoline -2-carboxamide or a salt thereof.

8. 7-(1-Methylethyl) -5-oxo-N-[2-(4-pyridinyloxy)butyl] -5H-thiazolo[2,3-b]quinazoline -2-carboxamide or a salt thereof.

9. N-[4-(lH-Imidazol-l-yl)butyl] -7-(1-methylethyl)-5--oxo-5H-thiazolo[2,3-b]quinazoline -2-carboxamide or a salt thereof.

10. A compound in accordance with any one of claims 1 to 9 for use as pharmaceutically active substance.

11. A compound in accordance with any one of claims 1 to 9 for use as anti-allergic agent or agent for the treatment of vascular disorders.

12. A process for the manufacture of compounds of formula I and of their pharmaceutically acceptable acid addition salts in accordance with any one of claims 1 to 9, which comprises
a) reacting an acid chloride of the general formula 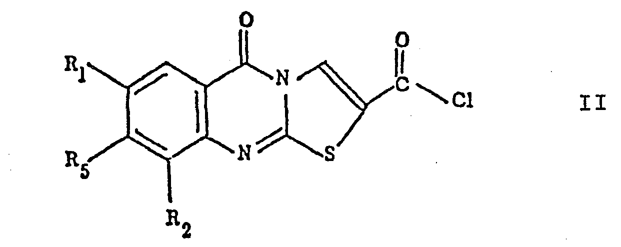 wherein R₁, R₂ and R₅ are as described in claim 1, with a compound of the general formula 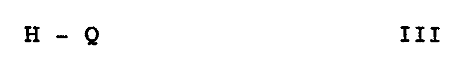 wherein Q is as described in claim 1, or
b) for the manufacture of a compound of formula I wherein _{Q} is the group (a) or (b), reacting an acid of the general formula 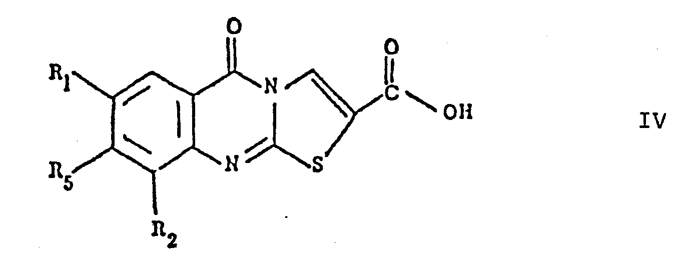 wherein R₁, R₂ and R₅ are as described in claim 1, with a compound of the general formula 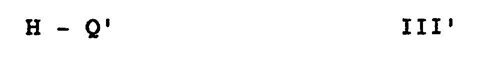 wherein Q' is the group (a) or (b), and, if desired, converting a compound obtained into a pharmaceutically acceptable acid addition salt.

13. A medicament containing a compound in accordance with any one of claims 1 to 9 or a pharmaceutically acceptable acid addition salt thereof and a therapeutically inert excipiens.

14. An anti-allergic agent or an agent for the treatment of vascular disorders containing a compound in accordance with any one of claims 1 to 9 or a pharmaceutically acceptable acid addition salt thereof and a therapeutically inert excipiens.
